# EUROPÄISCHE PATENTSCHRIFT

(11) **EP 2 303 142 B1**
(45) Veröffentlichungstag und Bekanntmachung des Hinweises auf die Patenterteilung: **25.01.2012**
(21) Anmeldenummer: 09736552.2
(22) Anmeldetag: 17.07.2009
(51) Int. Cl.: A61B 17/02, A61F 2/46

(54) **DISTRAKTIONSELEMENT FÜR DIE WIRBELSÄULE**
DISTRACTION ELEMENT FOR THE SPINAL COLUMN
ÉLÉMENT DE DISTRACTION POUR COLONNE VERTÉBRALE

(43) Veröffentlichungstag der Anmeldung: 06.04.2011
(73) Patentinhaber: Ulrich GmbH & Co. KG, 89081 Ulm (DE)
(72) Erfinder: PISCHL, Susanne, 89073 Ulm (DE); SCHMIDT, Thorsten, 89278 Nersingen (DE); WILLMANN, Nikolas, 89077 Ulm (DE)
(74) Vertreter: Hentrich, Swen
(86) Internationale Anmeldenummer: PCT/DE2009/075038
(87) Internationale Veröffentlichungsnummer: WO 2011/006456

(56) Entgegenhaltungen:
- EP-A1- 1 481 654
- WO-A1-2007/065126
- WO-A2-2006/090380
- US-A- 6 159 215
- US-A1- 2005 021 042

## Beschreibung

Die vorliegende Erfindung betrifft ein Distraktionselement für die Wirbelsäule und insbesondere betrifft sie ein Distraktionselement für die Wirbelsäule mit einer ersten zur Anlage an einem Wirbel angepassten Anlageplatte und einer zweiten zur Anlage an dem Gegenwirbel angepassten Anlageplatte.

Ein Distraktionselement der eingangs genannten Art ist aus der WO 2006/090380 A2 bekannt, wobei ein "Aktivator" zur Verstellung der Abstände zwischen der ersten und zweiten Anlageplatte mittels eines Scherenhebels vorgesehen ist.

Aus dem Stand der Technik sind bereits verschiedene Distraktionselemente für die Wirbelsäule bekannt. Allerdings weisen die vorbekannten Distraktionselemente den Nachteil einer umständlichen Handhabbarkeit für den Chirurgen auf.

Der Erfindung liegt daher die Aufgabe zugrunde, ein Distraktionselement für die Wirbelsäule der eingangs genannten Art so auszubilden, dass während der Operationen eine verbesserte Handhabung des Distraktionselementes erreicht werden kann.

Nach einem ersten Gesichtspunkt der vorliegenden Erfindung wird diese Aufgabe bei einem Distraktionselement für die Wirbelsäule mit einer ersten zur Anlage an einem Wirbel angepassten Anlageplatte und einer zweiten zur Anlage an dem Gegenwirbel angepassten Anlageplatte dadurch gelöst, dass ein Schlitten über einen Scherenhebel mit der ersten Anlageplatte verbunden und zur Verstellung eines Abstandes zwischen der ersten Anlageplatte und der zweiten Anlageplatte über eine Gewindespindel verfahrbar ausgestaltet ist, wobei die zweite Anlageplatte als eine Schiene für den Schlitten ausgestaltet ist. Diese Ausgestaltung weist den Vorteil auf, dass auf einfache Weise die Distraktion der Wirbel exakt eingestellt werden kann.

Bevorzugt weist die Gewindespindel ein Kopplungsteil auf, das zum Ankoppeln eines Einsetzinstrumentes ausgestaltet ist. Dadurch kann mit dem Einsetzinstrument das Distraktionselement besser bedient werden. Als vorteilhaft hat es sich erwiesen, wenn das Kopplungsteil Kopplungsteilnuten aufweist, die zum Eingriff für Zähne des Einsetzinstrumentes ausgebildet sind, und als besonders vorteilhaft hat es sich erwiesen, wenn die Kopplungsteilnuten als eine Vielzahnkupplung, insbesondere als ein Kronenrad ausgestaltet sind.

Bei einem bevorzugten Ausführungsbeispiel des Distraktionselements nach der vorliegenden Erfindung weist die erste Anlageplatte eine Abschrägung an einem von dem Kopplungsteil abgewandten und gegenüber liegenden Bereich auf. Dadurch wird die Einführung des Distraktionselements zwischen die Wirbelkörper der Wirbelsäule erleichtert.

Weiterhin kann bei dem Distraktionselement an einer Längsseite, insbesondere an beiden Längsseiten der zweiten Anlageplatte eine Führungsnut zur Führung eines Halters des Einsetzinstrumentes ausgebildet sein, wodurch die Stabilität gegenüber Beanspruchungen erhöht wird.

Darüber hinaus kann die erste Anlageplatte zur Aufnahme eines zur Anlage an einem Wirbel angepassten Anlageaufsatzes ausgestaltet sein, so dass der erzielbare Distraktionsbereich mittels verschieden hoher Aufsätze flexibel variiert werden kann.

Des Weiteren kann bei dem erfindungsgemäßen Distraktionselement der Schlitten zur Führung in einer Schwalbenschwanzführung auf der zweiten Anlageplatte ausgestaltet sein, wodurch eine gleichmäßigere und schonendere Distraktion ermöglicht wird. Außerdem kann die erste Anlageplatte eine Führungsöffnung zur Aufnahme eines Führungsstabes des Schlittens umfassen. Diese Ausgestaltung weist den Vorteil einer erhöhten Stabilität auf.

Nach einem zweiten Gesichtspunkt der vorliegenden Erfindung wird diese Aufgabe bei einem Einsetzinstrument für eines der vorgenannten Distraktionselemente, dadurch gelöst, dass das Einsetzinstrument zur Kopplung an das Distraktionselement eine Hohlwelle und einen Antriebsstab aufweist, so dass die Handhabbarkeit verbessert wird.

Außerdem kann das Einsetzinstrument einen zum Halten der zweiten Anlageplatte ausgestatteten Halter aufweisen, wodurch die Stabilität erhöht wird. Weiterhin kann zur besseren Handhabbarkeit das Einsetzinstrument einen Fixierstab umfassen.

Nach einem dritten Gesichtspunkt der vorliegenden Erfindung wird diese Aufgabe bei einem Distraktionssystem durch eine Kombination eines der vorgenannten Distraktionselemente mit einem der vorgenannten Einsetzinstrumente gelöst.

Im Folgenden wird die Erfindung an einem in der Zeichnung dargestellten Ausführungsbeispiel näher erläutert; es zeigen:
- Fig. 1: eine schematische Perspektivdarstellung eines Distraktionselementes nach der vorliegenden Erfindung,
- Fig. 2: eine Darstellung der Kopplung des Distraktionselementes aus der Figur 1 mit einem Einsetzinstrument,
- Fig. 3a: eine schematische Übersichtsdarstellung einer erfindungsgemäßen Kombination des Distraktionselementes mit dem Einsetzinstrument in einer Distraktionsstellung, bei der die Anlageplatten am weitesten voneinander beabstandet sind, und
- Fig. 3b: eine vergrößerte Darstellung des Bereiches Z aus der Fig. 3a.

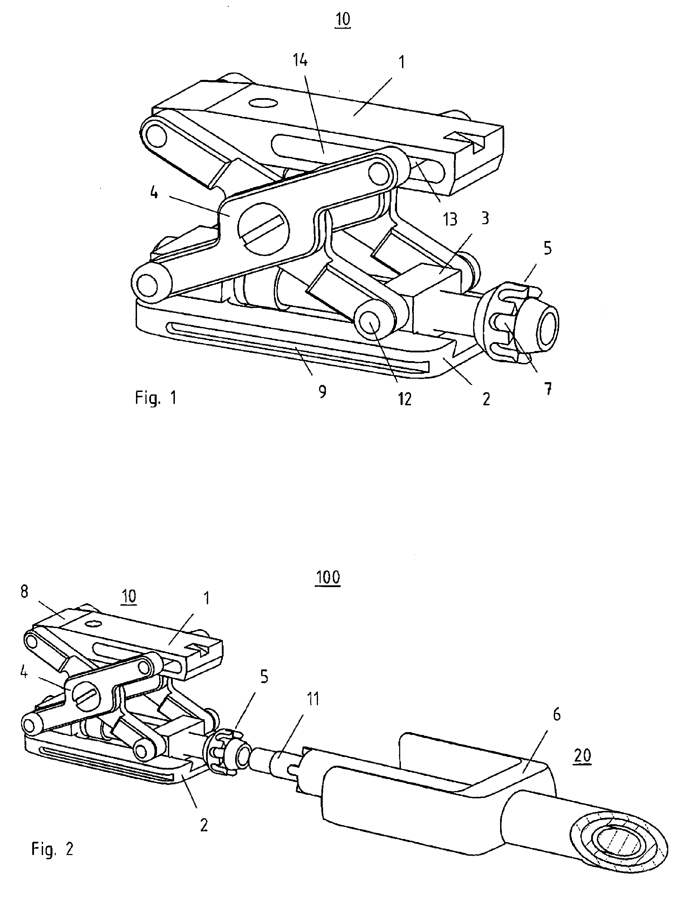

Eine schematische Perspektivdarstellung eines Distraktionselementes 10 für die Wirbelsäule ist in Fig. 1 gezeigt. Das Distraktionselement 10 umfasst eine erste zur Anlage an einem Wirbel angepasste Anlageplatte 1, eine zweite zur Anlage an dem Gegenwirbel angepasste Anlageplatte 2 und einen Schlitten 3. Der Schlitten 3 ist über einen Scherenhebei 4 mit der ersten Anlageplatte 1 verbunden und zur Verstellung eines Abstandes zwischen der ersten Anlageplatte 1 und der zweiten Anlageplatte 2 über eine Gewindespindel verfahrbar ausgestaltet, wobei die zweite Anlageplatte 2 als eine Schiene für den Schlitten 3 ausgestaltet ist. Auf diese Weise kann je nach Drehrichtung das Distraktionselement 10 distrahiert bzw. komprimiert werden.

Das Distraktionselement 10 kann einen Kopplungsteil 5 mit Kopplungsnuten 7 aufweisen. Weiterhin kann der Scherenhebel 4 einen Führungsstab 13 aufweisen, der beim Bewegen des Scherenbebels 4 in einer Führungsöffnung 14 der ersten Anlageplatte 1 geführt wird. Zusätzlich kann eine Führung des Schlittens 3 in einer Schwalbenschwanzführung auf der zweiten Anlageplatte 2 eine gleichmäßigere und schonendere Distraktion ermöglichen. Dabei dienen Scheiben 12 zur Befestigung des Schlittens 3 an dem Scherenhebel 4. Weiterhin kann die zweite Anlageplatte 2 eine Abschrägung 8 an dem von dem Kopplungsteil 5 abgewandten und gegenüber liegenden Ende aufweisen.

Fig. 2 zeigt eine schematische Darstellung der Kopplung des Distraktionselementes 10 mit einem Einsetzinstrument 20. Zur Kopplung wird das Distraktionselement 10 mit Hilfe eines Fixierstabes 11 an den Halter 6 angedockt. Dabei greifen Angriffszähne des Einsetzinstrumentes 20 in Kopplungsteilnuten 7 eines Kopplungsteiles 5 des Distraktionselementes 10 ein. Anschließend kann das Distraktionselement 10 mittels des Halters 6 in den Zwischenwirbelraum eingebracht werden. Mit dem Einsetzinstrument 20 kann das Distraktionselement 10 bis zur gewünschten Distraktion gezielt aufgespreizt werden. Durch ein Lösen des Fixierstabes 11 kann der Halter 6 von dem Distraktionselement 10 getrennt werden und das Distraktionselement 10 in situ verbleiben. Nach der Operation kann das Distraktionselement 10 wieder an den Halter 6 angedockt und durch den Fixierstab 11 gehalten werden. Durch ein Zurückdrehen des Einsetzinstrumentes 20 kann das Distraktionselement 10 wieder komprimiert und dadurch aus dem Zwischenwirbelraum entnommen werden.

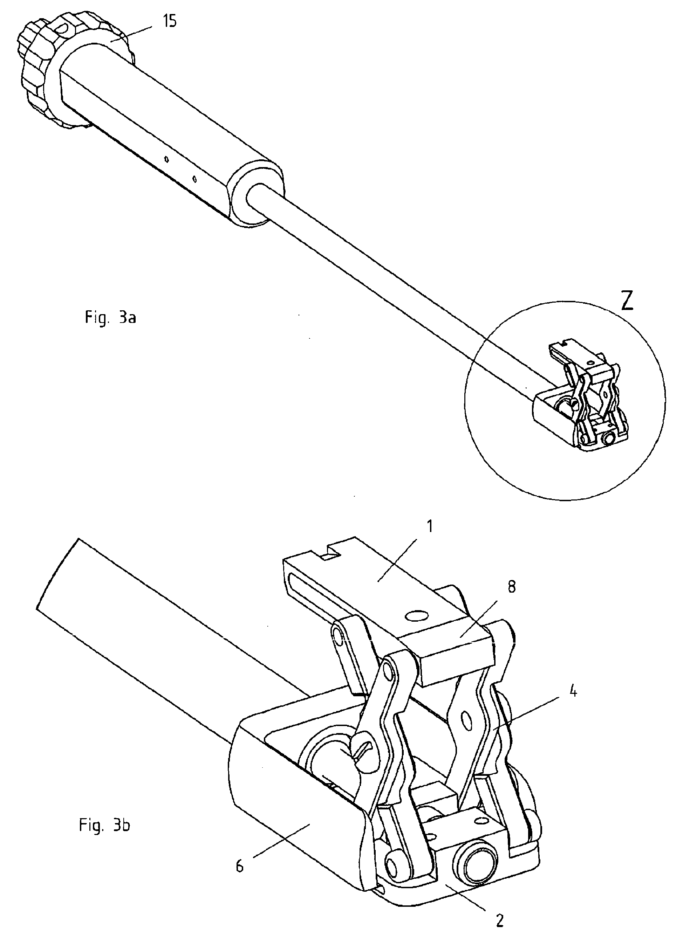

Fig. 3a zeigt zum besseren Verständnis eine Gesamtübersicht eines Distraktionssystems 100 mit dem Distraktionselement 10 und dem Einsetzinstrument 20. Mittels eines Stellrades 15 an dem Einsetzinstrument 20 kann die Distraktion gezielt gesteuert werden.

Fig. 3b zeigt eine detailierte und vergrößerte Darstellung des Bereiches Z aus der Fig. 3a in einer Stellung des Distraktionselementes 10, bei dem die erste Anlageplatte 1 von der zweiten Anlageplatte 2 maximal beabstandet ist. Um einen größeren Distraktionsbereich zu erzielen, können auf die zweite Anlageplatte 2 verschieden hohe Aufsätze angebracht werden.

### Bezugszeichenliste

- 1: erste zur Anlage an einem Wirbel angepasste Anlageplatte
- 2: zweite zur Anlage an einem Wirbel angepasste Anlageplatte
- 3: Schlitten
- 4: Scherenhebel
- 5: Kopplungsteil
- 6: Halter
- 7: Kopplungsteilnut
- 8: Abschrägung der zweiten Anlageplatte
- 9: Führungsnut zur Führung des Halters
- 10: Distraktionselement
- 11: Fixierstab
- 12: Befestigungsscheiben des Schlittens
- 13: Führungsstab des Schlittens
- 14: Führungsöffnung in der ersten Anlageplatte
- 15: Stellrad
- 20: Einsetzinstrument
- 100: Distraktionssystem

## Patentansprüche

1. Distraktionselement (10) für die Wirbelsäule mit einer ersten zur Anlage an einem Wirbel angepassten Anlageplatte (1), einer zweiten zur Anlage an dem Gegenwirbel angepassten Anlageplatte (2) und einem Schlitten (3), wobei der Schlitten (3) über einen Scherenhebel (4) mit der ersten Anlageplatte (1) verbunden und zur Verstellung eines Abstandes zwischen der ersten Anlageplatte (1) und der zweiten Anlageplatte (2) über eine Gewindespindel verfahrbar ausgestaltet ist und die zweite Anlageplatte (2) als eine Schiene für den Schlitten (3) ausgestaltet ist.

2. Distraktionselement (10) nach Anspruch 1, **dadurch gekennzeichnet, dass** die Gewindespindel ein Kopplungsteil (5) aufweist, das zum Ankoppeln eines Einsetzinstrumentes (20) ausgestaltet ist.

3. Distraktionselement (10) nach Anspruch 2, **dadurch gekennzeichnet, dass** das Kopplungsteil (5) Kopplungsteilnuten (7) aufweist, die zum Eingriff für Zähne (8) des Einsetzinstrumentes (20) ausgebildet sind.

4. Distraktionselement (10) nach Anspruch 3, **dadurch gekennzeichnet, dass** die Kopplungsteilnuten (7) als eine Vielzahnkupplung ausgestaltet sind.

5. Distraktionselement (10) nach einem der Ansprüche 2 bis 4, **dadurch gekennzeichnet, dass** die erste Anlageplatte (1) eine Abschrägung (8) an einem von dem Kopplungsteil (5) abgewandten und gegenüberliegenden Bereich aufweist.

6. Distraktionselement (10) nach einem der Ansprüche 2 bis 5, **dadurch gekennzeichnet, dass** an einer Längsseite, insbesondere an beiden Längsseiten der zweiten Anlageplatte (2) eine Führungsnut (9) zur Führung eines Halters (6) des Einsetzinstrumentes (20) ausgebildet ist.

7. Distraktionselement (10) nach einem der vorhergehenden Ansprüche, **dadurch gekennzeichnet, dass** die erste Anlageplatte (1) zur Aufnahme eines Anlageaufsatzes ausgestaltet ist.

8. Distraktionselement (10) nach einem der vorhergehenden Ansprüche, **dadurch gekennzeichnet, dass** der Schlitten (3) in einer Schwalbenschwanzführung geführt ist.

9. Distraktionselement (10) nach einem der vorhergehenden Ansprüche, **dadurch gekennzeichnet, dass** die erste Anlageplatte (1) eine Führungsöffnung (14) zur Aufnahme eines Führungsstabes (13) des Schlittens (3) aufweist.

10. Einsetzinstrument (20), **dadurch gekennzeichnet, dass** das Einsetzinstrument (20) zur Kopplung an ein Distraktionselement (10) nach einem der Ansprüche 1 bis 9 eine Hohlwelle und einen Antriebsstab aufweist.

11. Einsetzinstrument (20) nach Anspruch 10, **dadurch gekennzeichnet, dass** das Einsetzinstrument (20) einen zum Halten der zweiten Anlageplatte (2) ausgestalteten Halter (6) aufweist.

12. Einsetzinstrument (20) nach Anspruch 10 oder 11, **dadurch gekennzeichnet, dass** das Einsetzinstrument (20) einen Fixierstab (11) umfasst.

13. Kombination (100) eines Distraktionselementes (10) nach einem der Ansprüche 1 bis 9 mit einem Einsetzinstrument (20) nach einem der Ansprüche 10 bis 12.

## Claims

1. A distraction element (10) for the spinal column comprising a first contact plate (1) adapted to bear against a vertebra, a second contact plate (2) adapted to bear against the opposite vertebra, and a carriage (3), wherein the carriage (3) is connected to the first contact plate (1) by way of a scissor lever (4) and is displaceable by way of a threaded spindle for adjustment of the spacing between the first contact plate (1) and the second contact plate (2) and the second contact plate (2) is in the form of a rail for the carriage (3).

2. A distraction element (10) according to claim 1 **characterised in that** the threaded spindle has a coupling portion (5) adapted for coupling an insertion instrument (20) thereto.

3. A distraction element (10) according to claim 2 **characterised in that** the coupling portion (5) has coupling groove portions (7) adapted for engagement for teeth (8) of the insertion instrument (20).

4. A distraction element (10) according to claim 3 **characterised in that** the coupling groove portions (7) are in the form of a multi-tooth coupling.

5. A distraction element (10) according to one of claims 2 to 4 **characterised in that** the first contact plate (1) has a bevel (8) at a region opposite to and remote from the coupling portion (5).

6. A distraction element (10) according to one of claims 2 to 5 **characterised in that** a guide groove (9) for guiding a holder (6) of the insertion instrument (20) is provided at a long side, in particular at both long sides, of the second contact plate (2).

7. A distraction element (10) according to one of the preceding claims **characterised in that** the first contact plate (1) is adapted to receive a contact attachment.

8. A distraction element (10) according to one of the preceding claims **characterised in that** the carriage (3) is guided in a dovetail guide.

9. A distraction element (10) according to one of the preceding claims **characterised in that** the first contact plate (1) has a guide opening (14) for receiving a guide bar (13) of the carriage (3).

10. An insertion instrument (20) **characterised in that** the insertion instrument (20) has a hollow shaft and a drive bar for coupling to a distraction element (10) according to one of claims 1 to 9.

11. An insertion instrument (20) according to claim 10 **characterised in that** the insertion instrument (20) has a holder for holding the second contact plate (2).

12. An insertion instrument (20) according to claim 10 or claim 11 **characterised in that** the insertion instrument (20) includes a fixing bar (11).

13. A combination (100) of a distraction element (10) according to one of claims 1 to 9 with an insertion instrument (20) according to one of claims 10 to 12.

## Revendications

1. Elément de distraction (10) pour colonne vertébrale, comprenant une première plaque d'appui (1) destinée à être appliquée sur une vertèbre, une seconde plaque d'appui (2) destinée à être appliquée sur la vertèbre opposée, et un coulisseau (3) qui est relié à la première plaque d'appui (1) par un levier d'articulation (4) et qui, pour régler la distance entre la première plaque d'appui (1) et la seconde plaque d'appui (2) est constitué de manière à pouvoir se déplacer par l'action d'une broche filetée, la seconde plaque d'appui (2) ayant la forme d'un rail pour le coulisseau (3).

2. Elément de distraction (10) selon la revendication 1, **caractérisé en ce que** la broche filetée présente une partie d'accouplement (5), conçue pour permettre d'accoupler un instrument d'insertion (20).

3. Elément de distraction (10) selon la revendication 2, **caractérisé en ce que** la partie d'accouplement (5) présente des rainures d'accouplement (7) conçues pour accueillir des dents (8) de l'instrument d'insertion (20).

4. Elément de distraction (10) selon la revendication 3, **caractérisé en ce que** les rainures d'accouplement (7) forment un accouplement polygonal.

5. Elément de distraction (10) selon une des revendications 2 à 4, **caractérisé en ce que** la première plaque d'appui (1) présente une partie chanfreinée (8) dans une zone éloignée et située à l'opposé de la partie d'accouplement (5).

6. Elément de distraction (10) selon une des revendications 2 à 5, **caractérisé en ce que** sur un côté longitudinal, en particulier sur chacun des deux côtés longitudinaux de la seconde plaque d'appui (2) est prévue une rainure de guidage (9) pour accueillir un support (6) de l'instrument d'insertion (20).

7. Elément de distraction (10) selon une des revendications précédentes, **caractérisé en ce que** la première plaque d'appui (1) est conçue pour accueillir un chapeau d'appui.

8. Elément de distraction (10) selon une des revendications précédentes, **caractérisé en ce que** le coulisseau (3) se déplace dans un guide en queue d'aronde.

9. Elément de distraction (10 selon une des revendications précédentes, **caractérisé en ce que** la première plaque d'appui (1) présente une ouverture de guidage (14) pour accueillir une barre de guidage (13) du coulisseau (3).

10. Instrument d'insertion (20), **caractérisé en ce qu'**il présente un arbre creux et une barre d'entraînement pour s'accoupler à un élément de distraction (10) selon une des revendications 1 à 9.

11. Instrument d'insertion (20) selon la revendication 10, **caractérisé en ce qu'**il présente un support (6) conçu pour maintenir la seconde plaque d'appui (2).

12. Instrument d'insertion (20) selon la revendication 10 ou 11, **caractérisé en ce qu'**il comprend une barre de fixation (11).

13. Combinaison (100) d'un élément de distraction (10) selon une des revendications 1 à 9 et d'un instrument d'insertion (20) selon une des revendications 10 à 12.
